# EUROPÄISCHE PATENTANMELDUNG

(11) **EP 4 292 660 A1**
(43) Veröffentlichungstag der Anmeldung: **20.12.2023**
(21) Anmeldenummer: 22178784.9
(22) Anmeldetag: 13.06.2022
(51) Int. Cl.: A61P 35/00, A61K 9/00, A61K 9/16

(54) **SYSTEM ZUR VERABREICHUNG EINES WIRKSTOFFS**

(71) Anmelder: Fortis GmbH, 6060 Hall in Tirol (AT)
(72) Erfinder: FORSTER, Michael, 6060 Hall in Tirol (AT); HOLD, Dietmar, 8704 Herrliberg (CH); STEINMÜLLER, Thomas, 6060 Hall in Tirol (AT)
(74) Vertreter: Schwarz & Partner Patentanwälte GmbH

(57) **Zusammenfassung**

System (1) zur Verabreichung eines Wirkstoffs, umfassend einen magnetisierbaren Kern (2), wobei der Kern (2) eine Ummantelung (3) aus Siliziumdioxid aufweist, dadurch gekennzeichnet, dass die Ummantelung (3) den Kern (2) vollständig umschließt, wobei der Kern (2) mit Ummantelung (3) einen Durchmesser (D2) von 10 nm bis 400 nm, vorzugsweise 20 nm bis 250 nm, besonders bevorzugt 100 nm bis 200 nm aufweist, wobei auf der Ummantelung (3) eine Beschichtung (4) aufgebracht ist.

## Beschreibung

Die vorliegende Erfindung betrifft ein System zur Verabreichung eines Wirkstoffs. Weiters betrifft die Erfindung ein Verfahren zur Herstellung eines Systems zur Verabreichung eines Wirkstoffs.

### HINTERGRUND DER ERFINDUNG

Während die lokale Verabreichung von Wirkstoffen an der Oberfläche des Körpers relativ einfach ist, stellt die gezielte Verabreichung von Wirkstoffen an einen spezifischen Ort im Inneren des Körpers immer noch eine Herausforderung dar. Der häufigste Ansatz ist nach wie vor die systemische Verabreichung durch Verteilung eines Wirkstoffs über das Blut. Durch die Zirkulation im Blut verteilt sich der Wirkstoff über den gesamten Körper.

Zur gezielteren Verabreichung von Wirkstoffen werden inzwischen auch magnetisierbare Nanopartikel mit einer Wirkstoffbeschichtung injiziert. Zum Beispiel offenbart US 9,393,396 B2 ein Verfahren und eine Zusammensetzung von beschichteten Nanopartikeln zur lokalen hyperthermischen Behandlung von Tumorzellen. Die mit einem Wirkstoff angereicherten Nanopartikel werden mit Hilfe eines Magnetfeldes an eine definierte anatomische Stelle im Körper geleitet, wo die Nanopartikel den Wirkstoff abgeben können.

US 7,309,316 B1 beschreibt superparamagnetische Nanopartikel mit einem Rezeptor für spezifische Körperzellen, die mit einer Biopsie-Nadel in den Bereich einer ausgewählten Gewebeansammlung eingebracht werden. Ein magnetisierbarer bzw. magnetischer Stab wird durch die Kanüle der Biopsie-Nadel in den Wirkungsbereich der Nanopartikel eingeführt und dort für eine vordefinierte Zeit positioniert. Die Nanopartikel heften sich während einer definierten Behandlungsdauer an die Zellen. Nach Ablauf der Behandlungsdauer können die Nanopartikel mit anhaftenden Zellen aufgrund der magnetischen Wirkung kontrolliert mit dem magnetisierten bzw. magnetischen Stab zur weiteren Analyse entnommen werden.

US 10,449,160 B2 beschreibt intravenös zugeführte magnetisierbare Nanopartikel, die sich ohne äußeres Zutun (zufällig) in den neu gebildeten (neoangiogenen) Blutgefäßen um ein Tumorgewebe anreichern und mit ihrer (zufälligen) räumlichen Verteilung den Tumor umschließen. Die magnetisierbaren Nanoteilchen können mit einem Wirkstoff beschichtet sein, müssen es aber nicht zwingend.

EP 2 920 298 B1 offenbart mit Silica ummantelte Nanopartikel.

### KURZBESCHREIBUNG DER ERFINDUNG

Im Stand der Technik verwendete Systeme zur exakten Abgabe eines Wirkstoffs erweisen sich immer noch als unzureichend hinsichtlich Effizienz und Wirksamkeit in der Behandlung. Dies liegt unter anderem darin, dass die Partikel im Körper nicht exakt genug positioniert werden können und außerdem den Wirkstoff nicht gezielt genug freigeben. Aufgabe der vorliegenden Erfindung ist daher die Bereitstellung eines Systems zur Verabreichung eines Wirkstoffs im Körper, welches exakter positionierbar ist und den Wirkstoff effizienter am gewünschten Ort freigibt.

Gelöst wird diese Aufgabe durch ein System zur Verabreichung eines Wirkstoffs, umfassend einen magnetisierbaren Kern, wobei der Kern eine Ummantelung aus Siliziumdioxid aufweist, dadurch gekennzeichnet, dass die Ummantelung den Kern vollständig umschließt, wobei der Kern mit Ummantelung einen Durchmesser von 10 nm bis 400 nm, vorzugsweise 20 nm bis 250 nm, besonders bevorzugt 100 nm bis 200 nm aufweist, wobei auf der Ummantelung eine Beschichtung aufgebracht ist.

Die Erfinder haben herausgefunden, dass es beim System einerseits wesentlich auf den Durchmesser von Kern mit Ummantelung ankommt, um das System exakt im Körper eines Patienten mit einem Magneten positionieren zu können. Andererseits ist beim System die Beschichtung wesentlich, die nämlich die Aufgabe hat, den pharmazeutisch aktiven Wirkstoff in ausreichender Konzentration aufzunehmen. Daher ist die Beschichtung zur Aufnahme eines pharmazeutisch aktiven Wirkstoffs ausgebildet oder sie umfasst einen pharmazeutisch aktiven Wirkstoff.

Der Durchmesser von Kern mit Ummantelung liegt im Bereich von 10 nm bis 400 nm, vorzugsweise 20 nm bis 250 nm, besonders bevorzugt 100 nm bis 200 nm. In Tierversuchen konnten Systeme mit Durchmessern von 10 bis 30 nm mit gutem Erfolg verwendet werden. Da jedoch für den menschlichen Organismus eine Partikelgröße von 10 nm unter Umständen als problematisch angesehen wird, werden Größen von 100 nm bis 200 nm für den Einsatz in der Humanmedizin und kleinere Größen in der Veterinärmedizin bevorzugt. Der Durchmesserbereich 100 nm bis 200 nm stellt außerdem ein optimales Verhältnis zwischen Größe der Oberfläche der Ummantelung für die Beschichtung und Positionierbarkeit des Systems mit Hilfe eines lokalisierten Magnetfeldes dar.

Speziell für die humanmedizinische Anwendung ist ein Durchmesser des Kerns mit Ummantelung von nicht unter 100 nm vorteilhaft, da Partikel mit Durchmesser unter 100 nm vom menschlichen Körper oft mit großer Verzögerung ausgeschieden werden können. Teilchen kleiner als 10 nm haben sich überhaupt als zellgängig erwiesen und könnten daher in Zellen eindringen, was im Rahmen der Erfindung vermieden werden soll.

Für die Verträglichkeit im Organismus ist außerdem eine vollständige Ummantelung des Kerns mit der Ummantelung aus Siliziumdioxid entscheidend. Es soll damit sichergestellt sein, dass der Kern nicht in Kontakt mit Körperflüssigkeiten bzw. körpereigenen Flüssigkeiten kommt.

Der magnetisierbare Kern ist bevorzugt superparamagnetisch oder ferritisch. Superparamagnetische Kerne weisen gegenüber ferritischen Kernen den zusätzlichen Vorteil auf, dass sie keine agglomerierten Aggregate bilden und vereinzelt vorliegend bleiben, sodass superparamagnetische Kerne bevorzugt sind.

In einer Ausführungsvariante ist vorgesehen, dass der magnetisierbare Kern ausgewählt ist aus der Gruppe bestehend aus Maghemit (*γ*-Fe₂O₃), Cobaltferrit (CoFe₂O₄) oder Mischungen daraus.

Die Ummantelung aus Siliziumdioxid weist bevorzugt Poren auf, da so die Beschichtung besser an der Ummantelung haftet. In dieser Ausführungsvariante ist daher bevorzugt vorgesehen, dass die Beschichtung in die Poren ragt. Die Tiefe der Poren sollte 95% der Dicke der Beschichtung nicht überschreiten, sodass der Kern unzugänglich für Körperflüssigkeiten ist.

Das Zusammenspiel der drei Parameter Kern/Ummantelung/Beschichtung ist wesentlich dafür, dass und wie lange der Wirkstoff im System gehalten werden kann und wie er am gewünschten Ort freigegeben wird. Dabei zeigte sich, wie bereits oben ausgeführt, dass kleinere oder größere Kerne mit der Ummantelung und Beschichtung ebenso nicht funktionieren, wie andere Ummantelungen oder Beschichtungen bei der richtigen Kerngröße. Die Beschichtung ist für die Einlagerung eines pharmazeutisch aktiven Wirkstoffs bedeutsam. Die Beschichtung soll also so ausgebildet sein, dass ein Wirkstoff in die Beschichtung einlagerbar ist bzw. dass ein Wirkstoff eingelagert ist bzw. dass die Beschichtung die Aufgabe des Wirkstoffs selbst übernimmt. Gleichzeitig soll die Einlagerung des Wirkstoffs nur so stark sein, dass der Wirkstoff im Körper an einem gewünschten Ort auch wieder abgegeben wird (und nicht im System verbleibt). Die Bindungsstärke zwischen Wirkstoff und Beschichtung soll also ausgewogen sein. Es hat sich gezeigt, dass Beschichtungen auf Saccharidbasis, d.h. Monosaccharide, Oligosaccharide oder Polymere mit Saccharideinheiten geeignete Beschichtungen sind. Während sich Monosaccharide und Oligosaccharide bei der Abgabe des Wirkstoffs meist vollständig auflösen, sodass nur mehr noch der Kern mit Ummantelung übrig bleibt, können Polymere gegebenenfalls an der Ummantelung haften bleiben und nur den Wirkstoff an die Umgebung abgeben. Die Schichtdicke der Beschichtung hängt von der zu verabreichenden Wirkstoffmenge und dem Wirkstoff ab, sollte aber nicht weniger als 5% des Gesamtradius des Systems betragen.

Der Durchmesser des Kerns beträgt bezogen auf den Durchmesser aus Kern mit Ummantelung vorzugsweise zumindest 50 % und vorzugsweise maximal 95%. Besonders bevorzugt beträgt der Durchmesser des Kerns bezogen auf den Durchmesser aus Kern mit Ummantelung 75 % bis 85%.

Hinsichtlich der Beschichtung sind verschiedene Arten von Sacchariden und Polymeren mit Saccharideinheiten denkbar. Als Saccharide kommen verschiedene Verbindungen wie Monosaccharide und Oligosaccharide in Frage. Als Monosaccharide können exemplarisch Hexosen, beispielsweise Gulose, Idose, Galaktose, Talose, Allose, Altrose, Glucose oder Mannose, genannt werden, wobei bei den Monosacchariden Glucose und Mannose bevorzugt sind. Auch Oligosaccharide wie z.B. Saccharose etc. kommen in Frage.

Polymere mit Saccharideinheiten sind ebenfalls geeignete Beschichtungen, wobei hier der Wirkstoff in der Regel in das Polymer eingelagert wird. Unter Polymeren mit Saccharideinheiten werden Polymere verstanden, die aus Monomeren zusammengesetzt sind, wobei zumindest ein Monomer ein Saccharid ist. Im Rahmen der Erfindung sind Polymere mit Saccharideinheiten insbesondere Polysaccharide wie vorzugsweise Homoglykane (z. B. Dextran, Glycogen, Stärke (Amylose und Amylopektin), Pektine, Chitin, Callose, Cellulose etc.) oder vorzugsweise Heteroglykane (z. B. Hyaluronsäure, Agarose, Alginat, Xanthan etc.), wobei insbesondere Hyaluronsäure gute Kompatibilität zeigte.

Die Beschichtung kann einen Wirkstoff aufweisen. Dieser Wirkstoff kann in die Beschichtung eingebracht sein oder die Beschichtung selbst sein. Bevorzugt wird ein Wirkstoff allerdings erst kurz vor Anwendung in die Beschichtung eingebracht.

Typische Wirkstoffe sind z. B. solche für Krebstherapie, sodass der Wirkstoff z. B. ausgewählt sein kann aus der Gruppe bestehend aus Zytostatikum, Hormon, Antikörper, Zytokin, Signaltransduktions-Inhibitor, Proteaseinhibitor. Die Anwendung des Systems ist allerdings auch in der Schmerztherapie möglich, sodass der Wirkstoff z. B. ein Analgetikum sein kann. Auch ist die Anwendung des Systems nicht auf Humanbehandlung beschränkt, sondern auch in der Veterinärmedizin möglich.

Die Erfindung betrifft daher außerdem ein solches System zur Verwendung als Arzneimittel, z. B. in der Schmerztherapie, in der Onkologie, in der Veterinärmedizin etc.

Die Erfindung betrifft außerdem ein Arzneimittel, umfassend ein solches System und einen Wirkstoff. Dabei kann es sich um ein Arzneimittel, wie es z. B. in der Schmerztherapie, in der Onkologie, in der Veterinärmedizin etc. eingesetzt wird, handeln.

In einem Aspekt der Erfindung ist auch ein Verfahren zur Positionierung von Systemen der oben genannten Art vorgesehen, welches einen Stab, welcher ein Magnetfeld aufweist, umfasst, wobei der Stab an der Außenseite eines Körpers an der Haut positioniert wird.

Die Erfindung betrifft auch ein Verfahren zur Herstellung eines Systems zur Verabreichung eines Wirkstoffs. Das Verfahren umfasst die Schritte:
a) Bereitstellen von magnetisierbaren Kernen;
b) Ummanteln der Kerne mit einer Ummantelung aus Siliziumdioxid, beispielsweise mittels Sol-Gel-Verfahrens, so lange, bis die Dicke der Kerne mit Ummantelung zwischen 10 nm und 400 nm, vorzugsweise zwischen 20 nm und 250 nm, besonders bevorzugt zwischen 100 nm und 200 nm beträgt;
c) Beschichten der Ummantelung mit einer Beschichtung, welche geeignet ist, einen pharmazeutisch aktiven Wirkstoff aufzunehmen oder welche einen Wirkstoff darstellt.

Gegebenenfalls kann der Wirkstoff sofort oder später in die Beschichtung eingebracht werden.

Als Kerne kommen Partikel zum Einsatz, welche magnetisierbar sind und wie oben definiert aufgebaut sein können. Abhängig von der gewünschten Dicke aus Kern und Ummantelung erfolgt auch die Auswahl des Durchmessers der Kerne, wobei die Kerne vorzugsweise so ausgewählt sind, dass abhängig vom gewünschten Durchmesser von Kern samt Ummantelung die Kerne zumindest 50 % dieses Durchmessers ausmachen. Entsprechende vorteilhafte Durchmesser sind bereits oben definiert.

Die Beschichtung kann zuerst das Tränken des ummantelten Kerns mit einer wässrigen Lösung eines Saccharids oder eines Polymers mit Saccharideinheiten und das anschließende Trocknen zur Entfernung von Wasser umfassen.

Die Beschichtung der Ummantelung kann auch zuerst das Tränken des ummantelten Kerns mit einer Wirkstoff-Lösung und das anschließende Trocknen zur Entfernung von Wasser umfassen.

Der Wirkstoff wird bevorzugt als wässrige Lösung aufgebracht. Dabei ist es möglich, dass der Wirkstoff gemeinsam mit der Beschichtung aufgebracht wird, oder es kann der Wirkstoff nachträglich in die Beschichtung eingebracht werden (z. B. mittels Sprühtechnik eines gelösten oder suspendierten Wirkstoffs und anschließender Trocknung des Lösungsmittels oder Suspensionsmittel s).

Sollte die Beschichtung der Wirkstoff selbst sein, können analoge Techniken angewendet werden.

### DETAILLIERTE BESCHREIBUNG DER ERFINDUNG

Bei der gegenständlichen Erfindung handelt es sich um ein spezielles Verfahren zur minimalinvasiven, stark lokalisierten und personalisierbaren Behandlung von Tumoren oder anderer Läsionen im Körperinneren mittels geeigneter Wirkstoffe bei gleichzeitig maximaler Schonung daran angrenzender gesunder Gewebebereiche und minimalen Nebenwirkungen.

Die gegenständliche Behandlungsmethode beruht im Wesentlichen auf zwei Komponenten:
Als Komponente A (das System) fungieren magnetisierbare, biokompatible Nanopartikel mit einem superparamagnetischen (oder ferritischen) Kern, beispielswiese Maghemit (*γ*-Fe₂O₃) oder Cobaltferrit (CoFe₂O₄), ummantelt von porösem Silica. Die Poren der Siliziumdioxid-Ummantelung können eine Saccharid-Lösung, wie z. B. eine Glukose-Lösung, oder eine Lösung eines Polymers mit Saccharideinheiten, wie z.B. eine Hyaluronsäure-Lösung, als Beschichtung aufnehmen. Die adsorbierte Lösung (Beschichtung) enthält in Beispiel mindestens einen zur Tumorbehandlung pharmazeutisch aktiven Wirkstoff. Alternativ dazu kann die Beschichtung selbst der Wirkstoff sein.

Komponente B wird durch zumindest ein magnetisierbares bzw. magnetisches Objekt, vorzugsweise eines stabförmigen Permanent- oder Elektromagneten, repräsentiert. Die charakteristischen Abmessungen dieses Objekts hängen im Wesentlichen von der Ausdehnung der zu behandelnden Läsion ab. Die Geometrie und die Positionierung des zumindest einen Objekts wird von der gewählten (chirurgischen) Applikationstechnik beeinflusst. Die Effizienz der Behandlungsmethode wird gehoben, wenn sich das magnetisierbare bzw. magnetische Objekt im Nahebereich des zu behandelnden Bereichs oszillierend bewegt.

Während das System (Komponente A) in der Regel intraarteriell injiziert wird, erfolgt die Platzierung ein magnetisierbares bzw. magnetisches Objekt (Komponente B) z.B. durch Einführung über ein Trokar in der Nähe des gewünschten Ortes, an welchem das System positioniert werden soll. Die Positionierung des erfindungsgemäßen Systems kann aber auch mit einem magnetisierbaren bzw. magnetischen Objekt in Form eines Stabes erfolgen, welcher ein Magnetfeld aufweist, indem der Stab an der Außenseite eines Körpers an der Haut an der gewünschten Stelle positioniert wird.

Die Erfindung bietet sich vor allem für eine neuartige Tumorbehandlung an und sieht vorzugsweise die zeitgleiche und sehr präzise Applikation der beiden Komponenten im Nahebereich des zu behandelnden Gewebes (Komponente A wird intrakorporal, Komponente B wird intrakorporal oder an der Körperoberfläche positioniert) ausgehend von zwei unterschiedlichen Positionen vor. Die Zeitspanne zwischen der Applikation der beiden Komponenten kann auch eine definierte Dauer darstellen, entsprechend der Wirkstoffabgabe bzw. Wirkung des pharmazeutisch aktiven Wirkstoffs auf die Läsion zu optimieren.

Die magnetisierbaren Kerne mit Ummantelung und Beschichtung mit Medikation (Komponente A) werden über die Blutbahn (intraarterielle Applikation mittels Spritze oder über die Arterien mittels Katheder) in den Nahebereich des Tumors eingebracht und geben über einen definierten Zeitraum den Wirkstoff dosiert an das umgebende Gewebe ab. Das magnetisierbare bzw. magnetische Objekt (Komponente B) wird im Nahebereich der zu behandelnden Läsion beispielsweise mittels (magnetischer) Biopsie-Nadel, Trokar oder Katheder positioniert. Das applizierte Objekt (Komponente B) verbleibt für eine definierte Zeitspanne (von zumindest mehreren Sekunden) im Körper und kann wieder minimal-invasiv entfernt werden.

Aufgrund der magnetischen Wirkung zwischen eingebrachtem magnetisiertem bzw. magnetischem Objekt (Komponente B) und den magnetisierbaren Kernen verbleiben die Systeme (Komponente A) im Nahebereich der zu behandelnden Zellen (z.B. maligne Zellen im Falle eines Tumors) und können dort maximale Wirksamkeit erzielen. Begünstigt wird der Wirkstoffeintrag durch eine Bewegung des eingebrachten Objekts; diese Bewegung kann von außen über die Biopsie-Nadel, den Trokar, ein Katheder oder auf andere Art der Komponente B an der Körperoberfläche, erzwungen werden.

Das System verteilt sich nicht über einen weiten Bereich des Körpers und gewährleistet eine maximale Schonung gesunder Körperregionen. Aufgrund der stark lokalisierten Wirkung dieser Tumorbehandlung kann sie vereinfachend als "Chemotherapie ohne Nebenwirkung" verstanden werden.

Wenn die charakteristischen Abmessungen von Kern mit Ummantelung im Bereich 100 bis 200 nm liegt, sind diese nicht zellgängig und werden durch die hydrophile Silica-Ummantelung vor chemischen Angriffen körpereigener Substanzen geschützt; sie sind biokompatibel.

Nach Ende der Behandlungszeit können die magnetisierbaren Kerne mit Ummantelung wieder über ein magnetisches Hilfsmittel (z. B. Katheder mit Magnetspitze) gesammelt und schonend aus dem Körperinneren (rückstandslos) entfernt werden. Die nicht mit einem magnetischen Hilfsmittel entfernten Kerne mit Ummantelung werden allerdings auch nach einer gewissen Zeit automatisch ausgeschieden. Die Ausscheidung erfolgt in der Regel über das retikulohistiozytäre System.

Weitere Details und Vorteile der Erfindung sind in den beiliegenden Figuren und in der nachfolgenden Figurenbeschreibung dargelegt.
- Fig. 1: zeigt eine schematische Darstellung eines erfindungsgemäßen Systems.
- Fig. 2: zeigt die schematische Anwendung des Systems bei der Behandlung im Körper.

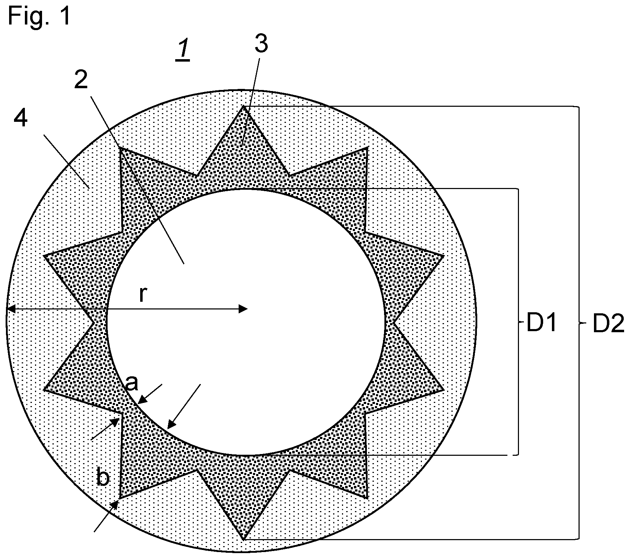

Fig. 1 zeigt grob schematisiert ein System 1 gemäß der Erfindung mit Radius r. Das System 1 zur Verabreichung eines Arzneimittels umfasst einen magnetisierbaren Kern 2, und zwar ist dieser entweder superparamagnetisch (er besteht in den untersuchten Beispielen aus Maghemit (*γ*-Fe₂O₃)) oder ferritisch (er besteht in den untersuchten Beispielen aus Cobaltferrit (CoFe₂O₄)) ausgebildet. Der superparamagnetische oder ferritische Kerns 2 weist einen Durchmesser D1 auf und wird von einer Ummantelung 3 aus Siliziumdioxid vollständig umgeben. Kern 2 samt Ummantelung 3 weisen einen Durchmesser D2 auf. Die Ummantelung 3 ist porös und weist Poren auf, die vereinfacht als dreieckförmige Einbuchtungen dargestellt sind. Die Tiefe der Poren sollte 95 % der Dicke b der Ummantelung 3 nicht unterschreiten, sodass eine Restdicke a von 5 % übrigbleibt.

Die Ummantelung 3 umschließt den Kern 2 vollständig. Auf der Ummantelung 3 befindet sich die Beschichtung 4, die im gezeigten Ausführungsbeispiel ein Saccharid oder ein Polymer mit Saccharideinheiten umfasst. Das Saccharid kann z. B. Glucose, die durch eine wässrige Glucoselösung auf die Ummantelung 3 aufgebracht wurde, sein. Auch Polymere wie Hyaluronsäure wurden in ähnlicher Art erfolgreich untersucht. Die Beschichtung 4 weist außerdem einen Wirkstoff auf, der mit der Saccharidlösung oder der Polymerlösung gleichzeitig aufgebracht wurde. Als Wirkstoff wurden Zytostatika, Hormone, Antikörper, Zytokine, Signaltransduktions-Inhibitoren, Proteaseinhibitoren und Analgetika untersucht. Es wäre auch denkbar, dass die Beschichtung 4 selbst ein Wirkstoff ist.

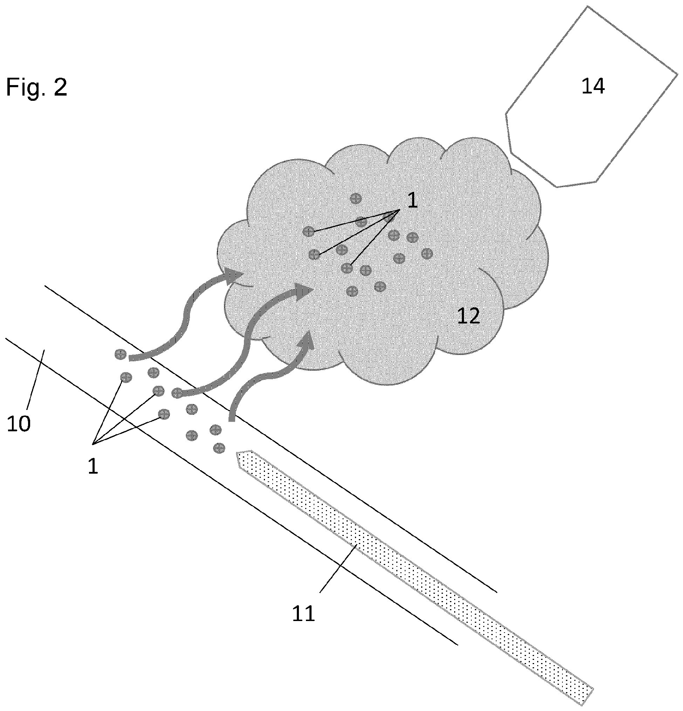

In onkologischen Untersuchungen wurden als Wirkstoff Zytostatika verwendet, die - wie in Fig. 2 - dargestellt mit dem System 1 über eine Spritze 11, einen Trokar, eine Biosie-Nadel oder einen Katheder in das Blutgefäß 10 injiziert wurden. Im Blutgefäß 10 verteilen sich die einzelnen Systeme 1 und werden in der Nähe eines Tumors 12 mittels eines Magnetfelds einer Magnetquelle 14 gelenkt und dort gehalten, wo sie den Wirkstoff zielgerichtet an den Tumor 12 abgeben und dort die zytostatische Wirkung entfalten können.

Ein erfindungsgemäßes System 1 wurde im Rahmen einer Studie an Kaninchen mit Tumor erfolgreich eingesetzt. Das untersuchte System 1 wies einen Kern 2 aus Cobaltferrit und eine Ummantelung aus Siliziumdioxid auf, der Durchmesser D2 von Kern 2 mit Ummantelung 3 aus Siliziumdioxid 12 bis 30 nm betrug und wobei der Durchmesser D1 des Kerns ca. 80 % des Durchmessers D2 betrug. Die Beschichtung 4 war in einem Fall aus Hyaluronsäure und im anderen Fall aus Glucose aufgebaut und die Schichtdicke der Beschichtung 4 betrug ca. 3 bis 4 nm. In die Beschichtung 4 war mittels Sprühtechnik ein Zytostatikum (Daunorubicin) als Wirkstoff eingebracht.

Mittels intraarterieller Injektion über die Arteria femoralis wurde das System 1 mit und ohne Wirkstoff (Kontrollgruppe 1) verabreicht. Es erfolgte eine einmalige Injektion von ca. 100 000 Systemen in 500 µl. Zum Zeitpunkt der Injektion befand sich bereits ein externes Magnetfeld im Bereich des Tumors. Mittels einmaliger Verabreichung erfolgte innerhalb von 14 Tagen eine Rückbildung an Tumorvolumen von 70 % bis 90 % bei der Patientengruppe, während bei der Kontrollgruppe 1 keine messbare Rückbildung an Tumorvolumen beobachtet werden konnte. In Kontrollgruppe 2 wurde das System 1 mit Wirkstoff injiziert, jedoch wurde kein externes Magnetfeld eingesetzt. Bei Kontrollgruppe 2 konnte eine Rückbildung an Tumorvolumen von 5 % bis 10 % beobachtet werden. Zwischen Beschichtung 4 aus Hyaluronsäure und solcher aus Glucose konnten keine signifikanten Unterschiede beobachtet werden, allerdings dürfte die Wirkstofffreisetzung bei der Beschichtung 4 aus Hyaluronsäure langsamer erfolgen.

## Patentansprüche

1. System (1) zur Verabreichung eines Wirkstoffs, umfassend einen magnetisierbaren Kern (2), wobei der Kern (2) eine Ummantelung (3) aus Siliziumdioxid aufweist, **dadurch gekennzeichnet, dass** die Ummantelung (3) den Kern (2) vollständig umschließt, wobei der Kern (2) mit Ummantelung (3) einen Durchmesser (D2) von 10 nm bis 400 nm, vorzugsweise 20 nm bis 250 nm, besonders bevorzugt 100 nm bis 200 nm aufweist, wobei auf der Ummantelung (3) eine Beschichtung (4) aufgebracht ist.

2. System nach Anspruch 1, **dadurch gekennzeichnet, dass** die Beschichtung (4) ein Saccharid oder ein Polymer mit Saccharideinheiten umfasst.

3. System nach Anspruch 1 oder Anspruch 2, **dadurch gekennzeichnet, dass** der magnetisierbare Kern (2) superparamagnetisch oder ferritisch ist.

4. System nach einem der Ansprüche 1 bis 3, **dadurch gekennzeichnet, dass** die Ummantelung (3) aus Siliziumdioxid Poren aufweist, wobei die Beschichtung (4) in die Poren ragt.

5. System nach einem der Ansprüche 1 bis 5, **dadurch gekennzeichnet, dass** der Durchmesser (D1) des Kerns (2) bezogen auf den Durchmesser (D2) aus Kern (2) mit Ummantelung (3) zumindest 50 %, vorzugsweise 75 % bis 85 % beträgt.

6. System nach einem der Ansprüche 2 bis 5, **dadurch gekennzeichnet, dass** die Beschichtung (4) ein Saccharid umfasst, wobei das Saccharid vorzugsweise ausgewählt ist aus der Gruppe bestehend aus Gulose, Idose, Galaktose, Talose, Allose, Altrose, Glucose und Mischungen daraus.

7. System nach einem der Ansprüche 2 bis 6, **dadurch gekennzeichnet, dass** die Beschichtung (4) ein Polymer mit Saccharideinheiten umfasst, wobei das Polymer vorzugsweise ausgewählt ist aus der Gruppe bestehend aus Hyaluronsäure, Dextran, Glycogen, Amylose, Amylopektin, der Pektine, Chitin, Callose, Cellulose und Mischungen daraus.

8. System nach einem der Ansprüche 1 bis 7, **dadurch gekennzeichnet, dass** die Beschichtung (4) einen Wirkstoff aufweist.

9. System nach Anspruch 8, **dadurch gekennzeichnet, dass** der Wirkstoff ausgewählt ist aus zumindest einem aus der Gruppe bestehend aus Zytostatikum, Hormon, Antikörper, Zytokin, Signaltransduktions-Inhibitor, Proteaseinhibitor, Analgetikum.

10. System nach einem der Ansprüche 1 bis 9, zur Verwendung als Arzneimittel.

11. System zur Verwendung nach Anspruch 10 zur Verwendung in der Humanmedizin oder Veterinärmedizin, insbesondere in der Schmerztherapie, in der Onkologie, in der Hämatologie und/oder in der Immunologie.

12. Arzneimittel, umfassend ein System nach einem der Ansprüche 1 bis 9 und einen Wirkstoff.

13. Verfahren zur Positionierung eines Systems nach einem der Ansprüche 1 bis 12, **dadurch gekennzeichnet, dass** ein magnetisches Objekt, vorzugsweise ein stabförmiges magnetisches Objekt, an der Außenseite eines Körpers an der Haut positioniert wird.

14. Verfahren zur Herstellung eines Systems (1) zur Verabreichung eines Wirkstoffs, insbesondere nach einem der Ansprüche 1 bis 12, umfassend die Schritte:
a) Bereitstellen von magnetisierbaren Kernen (2);
b) Ummanteln der Kerne (2) mit einer Ummantelung (3) aus Siliziumdioxid, vorzugsweise mittels Sol-Gel-Verfahren, so lange, bis die Dicke der Kerne mit Ummantelung (3) zwischen 10 nm und 400 nm, vorzugsweise zwischen 20 nm und 250 nm, besonders bevorzugt zwischen 100 nm und 200 nm beträgt;
c) Beschichten der Ummantelung (3), mit einer Beschichtung (4), welche geeignet ist, einen Wirkstoff aufzunehmen oder welche einen Wirkstoff darstellt.

15. Verfahren nach Anspruch 14, **dadurch gekennzeichnet, dass** die Beschichtung (2) der Ummantelung (3) zuerst das Tränken des ummantelten Kerns (2) mit einer wässrigen Lösung eines Saccharids oder eines Polymers mit Saccharideinheiten und das anschließende Trocknen zur Entfernung von Wasser umfasst.
